# EUROPEAN PATENT APPLICATION

(11) **EP 1 970 444 A1**
(43) Date of publication of application: **17.09.2008**
(21) Application number: 06834864.8
(22) Date of filing: 12.12.2006
(51) Int. Cl.: C12N 15/02, C12N 5/10, C12N 15/09, A61D 7/00

(54) **METHOD FOR PRODUCTION OF ANTIGEN-SPECIFIC HYBRIDOMA USING ARTIFICIAL LYMPH NODE WITH GOOD EFFICIENCY**

(30) Priority: 12.12.2005 JP 2005357949
(71) Applicant: Riken, Wako-shi, Saitama 351-0198 (JP)
(72) Inventor: WATANABE, Takeshi, RIKEN Yokohama Institute, Yokohama-shi, Kanagawa 2300045 (JP)
(74) Representative: Woods, Geoffrey Corlett
(86) International application number: PCT/JP2006/325137
(87) International publication number: WO 2007/069755

(57) **Abstract**

Provided herein is a method for efficiently producing a hybridoma producing a specific antibody using an artificial lymph node.
The method for producing an antigen-specific monoclonal antibody, comprising the following steps of:
(a) immunizing a nonhuman animal with an antigen and then transplanting a polymeric biomaterial containing a stromal cell that produces a cytokine into the immunized nonhuman animal, thereby causing the nonhuman animal to form an artificial lymph node;
(b) collecting the artificial lymph node formed in step (a) and then transplanting the artificial lymph node into a nonhuman immunodeficient animal;
(c) immunizing the nonhuman immunodeficient animal into which the artificial lymph node is transplanted in step (b) with the antigen of step (a) above;
(d) collecting a splenocyte from the nonhuman immunodeficient animal immunized in step (c); and
(e) fusing the splenocyte collected in step (d) with a myeloma cell to form an antibody-producing hybridoma.

## Description

### Technical Field

The present invention relates to a method for producing an antigen-specific hybridoma using an artificial lymph node.

### Background Art

To prepare a monoclonal antibody, an animal, such as a mouse, is immunized with an antigen and then antigen-producing plasma cells are generated. Subsequently, a group of spleen cells containing a high number of antigen-producing plasma cells are collected from the immunized animal. Hybridomas are formed by cell fusion of the spleen cells with myeloma cells. Many resultant hybridomas are screened for selection of the hybridomas producing a target antibody. The monoclonal antibody is produced by the thus obtained hybridomas (e.g., Koller G et al., Nature, 256, 495 (1975)).

Concerning preparation of monoclonal antibodies, various improvements have been performed to obtain higher quantities of hybridomas that produce a target antibody, for example, an elevation of cell fusion efficiency by applying an electric fusion method. However, when a mouse is used as an animal, one hybridoma is grown from 2 to 4 × 10⁴ splenocytes. Thus, at most only 500 hybridomas grow from one mouse. Therefore, it has been difficult to obtain a right suitable target hybridoma when only one out of 10⁵ or fewer cells producing a target specific antibody are present among splenocytes. As a fact, the numbers of specific-antibody-producing cells in the spleen differs depending on antigen type used for immunization. Hybridomas producing the right target-antibody generally account for only a few percent (%) of the hybridomas that have generally grown. It has been difficult to obtain monoclonal antibodies by a conventional hybridoma method, especially for an antigen with a small molecular weight, and proteins whose structures are relatively well conserved among species.

Lymph nodes are deeply involved in immunological function. A lymph node has a highly organized three-dimensional structure, where lymphocytes interact with antigens and antigen-presenting cells so as to initiate antigen-specific immunological responses. Lymph nodes are organs essential for biophylaxis against foreign antigens such as microorganisms. The present inventors have previously reported that artificial lymph nodes can be constructed via incorporation of stromal cells and cytokines into three-dimensional structures composed of polymeric bio-compatible materials followed by transplantation of them into mouse renal subcapsular spaces. The present inventors have shown that artificial lymph nodes can be constructed more efficiently by addition of activated dendritic cells derived from the bone marrow to the above combination (see JP Patent Publication (Kokai) No. 2004-255110 A).

### Disclosure of the Invention

The present invention provides a novel method for an efficient production of a hybridomas secreting a specific target antibody by applying the artificial lymph node method.

With the conventional cell fusion method, the resulting efficiencies for the formation of specific target antibody-producing hybridomas have not been always high. The present inventors have intensively examined a method for producing hybridomas through the combination with the previously developed method for construction of an artificial lymph node. Specifically, the present inventors have discovered that the efficiency of the formation of specific-antibody-producing hybridomas can be extraordinarily elevated by constructing artificial lymph nodes using activated dendritic cells (pulsed with an antigen within renal subcapsular spaces of mice to which the antigen has been administered), retransplanting the thus obtained artificial lymph nodes into immunodeficient mice, immunizing the immunodeficient mice with the antigen, collecting splenocytes, and thus producing hybridomas. Thus, the present inventors have completed the present invention.

The present invention is as described below.
[1] A method for production of an antigen-specific hybridoma, comprising the following steps of:
   (a) immunizing a nonhuman animal with an antigen and then transplanting a polymeric biomaterial containing a stromal cell that produces a cytokine into the immunized nonhuman animal, thereby causing the nonhuman animal to form an artificial lymph node;
   (b) collecting the artificial lymph node formed in step (a) and then transplanting the artificial lymph node into a nonhuman immunodeficient animal;
   (c) immunizing the nonhuman immunodeficient animal, into which the artificial lymph node is transplanted in step (b), with the antigen of step (a) above;
   (d) collecting the splenocytes from the nonhuman immunodeficient animal immunized in step (c); and
   (e) fusing the splenocytes collected in step (d) with a myeloma cell in order to make an antibody-producing hybridoma.
[2] The method of [1], in which the polymeric material further contains an activated dendritic cell pulsed with the antigen of step (a) in [1].
[3] The method of [1] or [2], in which the stromal cell is a TEL-2 cell.
[4] The method according to any one of [1] to [3], in which the polymeric material is a collagen sponge.
[5] The method according to any one of [1] to [4], in which the cytokine is lymphotoxin and/or chemokine.
[6] The method according to any one of [1] to [5], in which the nonhuman animal is a mouse.
[7] The method according to any one of [1] to [6], in which the immunodeficient animal is an immunodeficient mouse.
[8] The method according to any one of [1] to [7], in which the immunodeficient mouse is an SCID mouse or a RAG2 gene-deficient mouse.
[9] A kit for producing an antigen-specific hybridoma, containing:
   (a) a cytokine expression vector and a stromal cell or a stromal cell transfected with a cytokine expression vector;
   (b) a polymeric biomaterial; and
   (c) an activated dendritic cell.
[10] The kit for producing an antigen-specific hybridoma according to [9], further containing:
   (d) a mouse for artificial lymph node formation; and
   (e) an immunodeficient mouse.
[11] The kit for producing an antigen-specific hybridoma according to [9] or [10], in which an antigen is further contained so that an activated dendritic cell is pulsed with the antigen.

This description includes part or all of the contents as disclosed in the description and/or drawings of Japanese Patent Application No. 2005-357949, which is a priority document of the present application.

### Brief Description of the Drawings

Fig. 1 is a schematic diagram for construction of artificial lymph nodes.
Fig. 2 shows an outline of the steps of the method of the present invention, comprising construction of artificial lymph nodes and retransplantation of the artificial lymph nodes into immunodeficient mice, followed by obtainment of immunocytes.
Fig. 3 is a graph showing specific IgG antibody levels in mouse sera upon construction of artificial lymph nodes and under different conditions for immunization of SCID mice.
Fig. 4 shows the results of measuring the level of a specific antibody produced by the thus obtained hybridoma in the culture solution of each well.

### Best Mode for Carrying Out the Invention

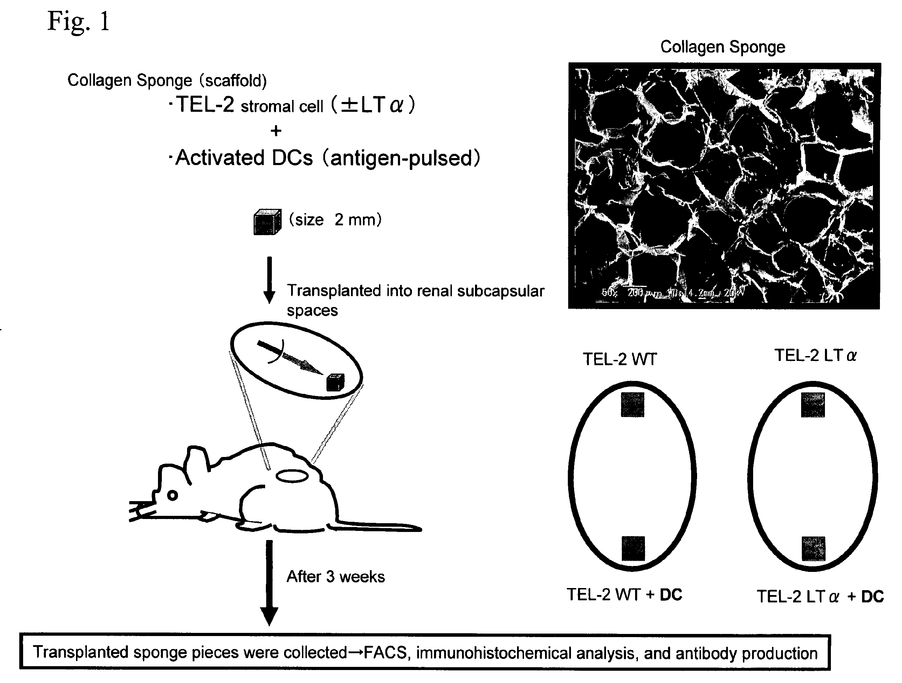

In the method of the present invention, artificial lymph nodes can be produced according to the method disclosed in JP Patent Publication (Kokai) No. 2004-255110 A. Fig. 1 is a schematic diagram showing the construction of artificial lymph nodes.

Lymph nodes are lymphoid tissues (the tissues in which lymphocytes interact with nonlymphoid cells) and are tissue organs that play an important role in maturation of lymphoid cells or adaptive immune response. Lymphoid tissues can be classified into primary lymphoid tissues and secondary lymphoid tissues. The primary lymphoid tissue is a site of lymphocyte production, and it includes the bone marrow and thymus tissue. Furthermore, the secondary lymphoid tissue has a special structure to capture antigens and is a site at which adaptive immune response is initiated. Examples of such secondary lymphoid tissue (also referred to as peripheral lymphoid tissue) include tissue of the spleen and lymph nodes, and mucosa-associated lymphatic tissues (tonsilla, trachea-associated lymphatic tissues, gut-associated lymphatic tissues, Peyer's patches (PP), and aggregates of other lymphoid cells).

An antigen-specific antibody-producing artificial lymph node that is used for production of the antigen-specific hybridoma of the present invention can be constructed by transplanting a combination of the following three elements into an animal:
(a) a cell that is an alternative for a stromal cell, which is important for lymph node formation;
(b) a cytokine, which is important for lymph node formation; and
(c) a three-dimensional scaffold composed of a biocompatible polymeric material.

A specific-antibody-producing artificial lymph node to be used for production of an antigen-specific hybridoma according to the present invention is provided with a basic structure of a lymph node that is considered to be essential as a location for initiation of an antigen-specific immunoreaction, as described below.
[1] A T cell domain and a B cell domain that are clearly distinguishable are present.
   Similarly to normal lymphoid tissue, a T cell domain and a B cell domain are clearly separated from each other in the specific antibody-producing artificial lymph node of the present invention, referred to as a "T cell domain" and a "B cell domain," respectively. In particular, a B cell population is also referred to as a "follicle."
[2] Dendritic cells playing an important role in immunoreaction are present together with T cells and B cells.
[3] A B cell domain containing a network of follicular dendritic cells is present in the central region.
   "Follicular dendritic cells (FDCs: follicular dendritic cells)" means "special cells having dendrites, which are present in the follicular central region." FDCs form a network in the follicular central region (referred to as the FDC network) and are of a cell type that differs from dendritic cells.
[4] Germinal center B cell-like B cells strongly positive for PNA (peanut agglutinin) are present.
   Prior to antibody production due to stimulation with an antigen, active proliferation of B cells within the follicular central region and differentiation into plasma cells (antibody-producing cells) take place. This site is referred to as the germinal center (GC). B cells that actively grow in the germinal center are referred to as germinal center B cells. Germinal center B cells have the property of frequently binding to PNA and thus are known to be strongly positive for PNA (PNA^{high+}) when they are stained with PNA.
[5] Plasma cells that are antibody-producing cells are present.
   Antibody-producing cells are B cells that have differentiated to reach the final stage and are referred to as plasma cells.
[6] An HEV-like vascular structure, which is a portal of entry for a lymphocyte into a lymph node, is present.

High endothelial venules (HEVs) are special vascular structures that are specifically observed in secondary lymphoid tissues such as lymph nodes and Peyer's patches. Unlike general blood vessels, HEVs contain tall (thick wall) endothelial cells. HEVs express some kinds of adhesion factor or chemokine and provide entries for lymphocytes that migrate via the bloodstream and enter such secondary lymphoid tissues.

The term "cytokine" is a generic term for proteinous physiologically active substances controlling the growth and differentiation of various types of blood cell, or this term may further refer to as a growth factor or a growth suppressing factor for cells including non-immune cells. Cytokines are classified based on the properties of their effects into interleukins, colony-stimulating factors, interferons, chemokines, lymphokines, tumor necrosis factors (TNFs), and the like. Interleukins that are used in the present invention are not particularly limited and can be arbitrarily selected from among IL-1 to IL-18. As a colony-stimulating factor, G-CSF, M-CSF, GM-CSF, or the like can be used, for example. As an interferon (IFN), IFN-α, IFN-β, IFN-γ, or the like can be used, for example. As a chemokine, CCL21 (also referred to as SLC (secondary lymphoid tissue chemokine)), CXCL13 (also referred to as BLC (B lymphocyte chemoattractant)), CCL19 (also referred to as ELC (Epstein-Barr virus-induced molecule 1 ligand chemokine)), or the like can be used, for example. As TNF, TNF-α, TNF-β, or the like can be used, for example. TNF-β is also referred to as lymphotoxin α (LTα). LTα is a first molecule found to be essential for the first time for organogenesis of lymph nodes (LNs) and Peyer's patches (PP) and for normal formation of splenic tissue structure. Lymphotoxins include LTβ in addition to LTα. These lymphotoxins can also be used in the present invention.

Cytokines to be used in the present invention are preferably lymphotoxins and/or chemokines, more preferably LTα, CCL21, CXCL13, and/or CCL19. These cytokines are commercially available and can be easily obtained.

"Stromal cell" is a generic term for cells composing microenvironments surrounding various cells (parenchymal cells) having unique functions specific to glands or organs. They are also referred to as "*Kanshitu saibo* (mesenchymal cells)." Such "*Kanshitu saibo* (mesenchymal cells)" are thought to physically support parenchymal cells, as indicated by the meaning of the Japanese term, and to exert support functions by which some effects are exerted on the other cell via cell-to-cell interaction. Stromal cells are also referred to as "supporting cells" or "interstitial cells."

Examples of stromal cells to be used in the present invention include TEL-2 stromal cells (Eur. J. Immunol 20: 47-53, 1990) established from 2-week-old Balb/c mouse thymus. The TEL-2 cells can be maintained and subcultured by culturing the cells in RPMI-1640 medium supplemented with inactivated 10% FCS and 50 µM 2-mercaptoethanol. For example, cells can be subcultured by collecting the cells (being cultured) every 3 days from a culture dish using a Trypsin-EDTA solution and then diluting the resultant to 1/10 to 1/20.

Stromal cells producing a cytokine used in the present invention can be prepared by constructing an expression vector (also referred to as a cytokine expression vector) containing a gene encoding the cytokine and then transfecting the stromal cells with the expression vector using a known gene transfer technique. Gene information concerning various cytokines is known and is available from publicly usable gene databases, such as GenBank.

For example, to cause production of the above cytokines or chemokines via TEL-2 stromal cells, expression vectors containing genes encoding them are introduced via a lipofection method or the like and then the cells are cultured in selection medium supplemented with G418 (500 µg/ml) for 10 days to 2 weeks. Thus, drug-resistant cell lines can be obtained. Expression of a transgene can be confirmed by measuring the biological activity of a cell culture supernatant. For measurement of biological activity, in the case of a chemokine, chemotaxis assay, which is used for measuring chemotactic activity to T cells or B cells, can be used, for example.

As a polymeric biomaterial to be used in the present invention, a biocompatible polymeric material having a three-dimensional structural skeleton can be used. In the present invention, a "three-dimensional structural skeleton" means a "scaffold" that allows stromal cells and cells composing lymph nodes, such as lymphocytes and dendritic cells, to be able to organize three-dimensional tissues. Furthermore, a "biocompatible polymeric material" means "a material composed of various polymers, characterized in that when the material is applied to a living body by a relevant method, the material can suppress the organism's reaction for eliminating the applied material as a foreign substance to as low a level as possible."

Examples of such polymeric biomaterial include collagen, glycosaminoglycan, polyglycolic acid, and poly-L-lactic acid. Furthermore, non-biodegradable materials such as nylon, polyester, polyurethane, and ethylene vinyl acetate, can be used independently or used in appropriate combinations thereof.

A collagen sponge is a component of a living body and is preferable as a polymeric biomaterial since the collagen sponge can suppress inflammatory reaction or immunoreaction at a low level. "Collagen sponge" means a porous material having a sponge structure containing collagen. For example, collagen for three-dimensional tissue culture or the like can be used in the present invention, which is prepared by freeze-drying insoluble collagen of cattle Achilles tendon to obtain sponge-shaped porous collagen.

A polymeric biomaterial to be used in the present invention may be biodegradable or non-biodegradable. Any polymeric biomaterial can be used as long as it is a material that is unlikely to cause immunoreaction due to its own antigenicity and/or inflammatory reaction arising from physical stimulation when it is transplanted into a living body. However, for stromal cells and immunocompetent cells to be organized into a three-dimensional structure capable of functioning as an artificial lymph node, for example, in the case of a porous polymeric material, selection of a pore diameter (pore size) appropriate for construction of an artificial lymph node is required, or selection of the entire size of polymeric materials to be transplanted is required. The pore diameter or the size of such transplanted material can be adequately determined by persons skilled in the art.

The term "dendritic cell" is a generic term for cell groups having dendritic shape derived from hematopoietic stem cells. Dendritic cells are distributed not only in lymphoid organs, but also widely distributed in organs other than lymphoid organs. Dendritic cells play an important role in initiation of immune response as effective stimulating factors for T cells and B cells upon activation.

Dendritic cells to be used in the present invention can be obtained by culturing the precursor cells thereof. Precursor cells of dendritic cells are originated from hematopoietic stem cells derived from the bone marrow, umbilical cord blood, or peripheral blood, monocytic cells derived from peripheral blood, or the like. Precursor cells of dendritic cells can be separated and purified according to need from the suspension of a collected bone marrow aspirate, umbilical cord blood, peripheral blood, or the like.

An adequate inducer is used to grow a cell group including precursor cells of dendritic cells and thus to induce differentiation thereof into mature/activated dendritic cells. As inducers, cytokines can be selected and used. Examples of such cytokines include GM-CSF, IL-1, IL-4, IFN-α, and TNF-α and they can be used independently or in adequate combinations thereof.

For maturation/activation of dendritic cells, RPMI-1640 medium supplemented with the above cytokine (5 ng/ml to 20 ng/ml), McCoy's medium, or the like is used, for example. To finally cause activation of such cells, an activation factor or such as LPS (lipopolysaccharide) is added and then the cells are cultured.

Furthermore, in the method of the present invention, dendritic cells are preferably antigen-pulsed by culturing the cells after addition of an antigen against a monoclonal antibody to be prepared.

A specific-antibody-producing artificial lymph node to be used for production of the antigen-specific hybridoma of the present invention can be constructed by transfecting stromal cells with an expression vector containing a gene encoding an arbitrary cytokine, preparing cytokine-producing stromal cells, adhering the cytokine-producing stromal cells to a polymeric biomaterial, and then transplanting the polymeric biomaterial into a tissue of an animal immunized with an antigen against a monoclonal antibody to be prepared. Stromal cells can be adhered to a polymeric biomaterial by immersing stromal cells in a cell suspension prepared to have a high concentration or by injecting stromal cells into a polymeric biomaterial using a syringe provided with an injection needle (e.g., a 26-gauge needle).

Preferably, in addition to cytokine-producing stromal cells, dendritic cells can also be added to such a polymeric biomaterial. More preferably, activated dendritic cells that have been pulsed with an antigen (that is, an antigen against a monoclonal antibody to be prepared) same as that used for immunization of the animal can also be added.

Examples of animals to be subjected to transplantation of a polymeric biomaterial to which stromal cells (and activated dendritic cells) have been adhered include human and nonhuman animals. Nonhuman animals are preferably nonhuman mammals (e.g., monkeys, horses, cattle, goat, sheep, pigs, dogs, cats, rabbits, guinea pigs, hamsters, rats, and mice). Of these nonhuman mammals, mice that are generally used for hybridoma production are particularly preferable.

These animals are immunized with an antigen against a monoclonal antibody to be prepared before transplantation of a polymeric biomaterial. Because of this immunization, immunocompetent cells (e.g., B cells and T cells) within the animals' secondary lymphoid tissues and the like are antigen-stimulated, thereby generating antibody-producing cells (B cells) or antigen-specific T cells expressing the antibody specific to the antigen (preferably, IgG, IgA, and/or IgM) on cell surfaces and the like.

Examples of an antigen to be used for immunization include, but are not limited to, pathogenic microorganisms such as protozoans, fungi, bacteria, and viruses. Moreover, as antigens, proteins, peptides, nucleic acids, lipids, carbohydrates, and the like, which are associated with cancer or specific diseases, can also be used, in addition to these pathogenic microorganisms.

Upon immunization, these antigens are preferably administered together with an adjuvant (e.g., alum, Freund's complete adjuvant, or incomplete adjuvant) to an animal.

The amount of an antigen to be used for immunization of an animal in which lymph nodes are constructed differs depending on the type, age, and body weight of an animal to be used herein, the route of administration, and the like. For example, in the case of an adult mouse, 10 µg to 1000 µg and preferably 10 µg to 100 µg of an antigen precipitated in alum may be administered intraperitoneally.

Furthermore, to ensure stimulation of immunocompetent cells (e.g., B cells) with an antigen, primary immunization may be repeated at intervals of 1 to 2 weeks or one or several times (e.g., 1 to 3 times). When immunization is repeated, an antigen is preferably administered intravenously (i.v.).

About 3 weeks after transplantation of a polymeric biomaterial, specific-antibody-producing artificial lymph nodes to be used for antigen-specific hybridoma production of the present invention can be constructed within the animal's body. The thus constructed specific-antibody-producing artificial lymph nodes are collected from the living body. The thus collected artificial lymph nodes can be stored in a culture solution supplemented with an appropriate preservative (e.g., 10% DMSO) at a low temperature (-80°C or less), prepared before use, and then used.

Such collected artificial lymph nodes are retransplanted into a recipient immunodeficient animal. The immunodeficient animal to be used herein is preferably an animal of the same species as the animal used for construction of the artificial lymph nodes. Sites for transplantation may be similar to those into which a polymeric biomaterial containing stromal cells and the like is transplanted upon construction of artificial lymph nodes. For example, artificial lymph nodes are transplanted into renal subcapsular spaces.

An immunodeficient animal, into which collected artificial lymph nodes are retransplanted, may be an immunodeficient animal lacking functional T and B lymphocytes, such as an immunodeficient mouse. As immunodeficient mice, nude mice, SCID mice (severe combined immunodeficiency mice), RAG2 -gene-deficient mice, and the like can be used. Examples of SCID mice include NOD/Shi mice, C.B-17/IcrCrj-scid/scid mice, and NOG mice (NOD/Shi-scid/IL-2γ -/- mice). These SCID mice are available from CLEA Japan, Inc.

When retransplantation is performed, 2 to 4 artificial lymph nodes are preferably transplanted. Specifically, 1 to 2 artificial lymph nodes are transplanted into subcapsular spaces of the kidney on each side.

After artificial lymph nodes are transplanted into an immunodeficient animal, the immunodeficient animal is immunized with an antigen that is used for construction of the artificial lymph nodes. At this time, immunization may be performed more than once. For example, immunization may be performed after 1 day to 2 weeks and preferably after 1 to 2 days, and then immunization may be further performed after 1 to 2 weeks and preferably after 1 week to 10 days. Preferably, immunization is performed twice (primary immunization and secondary immunization). An immunization method to be employed herein is desirably performed via intravenous administration. The dose of an antigen ranges from, in the case of a mouse, 10 µg to 1000 µg, and it preferably ranges from 10 µg to 100 µg. The dose employed in the second immunization may be lower than that used for the first immunization. For example, an antigen may be administered in an amount that is one-tenth of the dose of the 1^{st} immunization. B lymphocytes that have been antigen-stimulated by primary immunization cluster in the transplanted artificial lymph nodes. Subsequently, the B lymphocytes are exposed to the same antigen as that used for primary immunization and then undergo a class switch in the artificial lymph nodes, so as to induce antibody-producing cells (cells producing IgG, IgE, or IgA). In the present invention, antibody-producing cells are preferably cells producing an IgG type antibody (e.g., IgG₁, IgG₂, IgG₃, or IgG₄).

Several days after and preferably 4 to 5 days after final immunization, immunocytes are collected from the mammal and then cell fusion is performed. At this time, splenocytes are preferably used as immunocytes. Furthermore, before cell fusion is performed, it is desirable to confirm an elevated level of the desired antibody in the immune serum of the immunodeficient animal by ELISA, Western blotting, or the like.

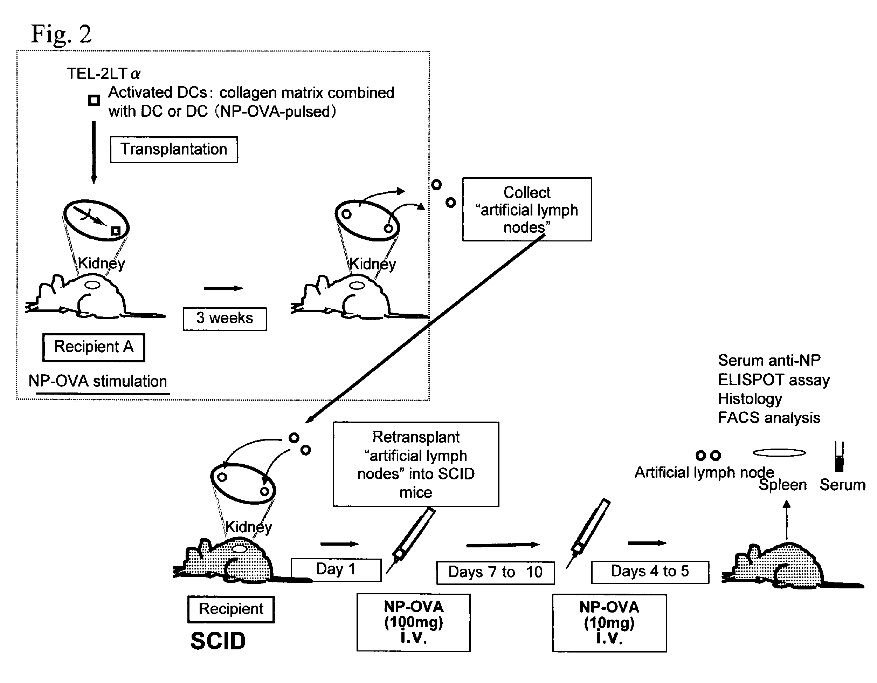

Fig. 2 shows an overview of the steps of the present invention, comprising construction of artificial lymph nodes, retransplantation into immunodeficient mice, and then obtainment of immunocytes.

The present invention further provides a method for producing a human specific-antibody-producing hybridoma. In this case, human specific-antibody-producing artificial lymph nodes to be used for production of such antigen-specific hybridoma can be constructed by, for example, introducing human lymphocytes, human lymphocyte precursor cells, bone marrow cells, and/or hematopoietic stem cells into an immunodeficient animal (e.g., an immunodeficient mouse) to construct a human immune system *in vivo* in the immunodeficient animal, immunizing the animal having the human immune system with an antigen against a monoclonal antibody to be prepared according to the above method, transplanting a polymeric biomaterial (artificial lymph node) to which stromal cells and/or dendritic cells have been adhered, and then performing secondary immunization with the same antigen according to need.

The thus constructed human specific-antibody-producing artificial lymph nodes are retransplanted into an immunodeficient animal, so that hybridomas producing the human monoclonal antibody can be produced.

Mammalian myeloma cells are used as cells to be fused to immunocytes collected from an immunodeficient animal. Examples of such myeloma cells that can be appropriately used herein include cells of various known cell lines such as, in the case of mouse-derived cells, P3 (P3x63Ag8.653) (J. Immnol. (1979) 123, 1548-1550), P3x63Ag8U.1 (Current Topics in Microbiology and Immunology (1978) 81, 1-7), NS-1 (Kohler. G. and Milstein, C. Eur. J. Immunol. (1976) 6, 511-519), MPC-11 (Margulies. D. H. et al., Cell (1976) 8, 405-415), SP2/0 (Shulman, M. et al., Nature (1978) 276, 269-270), FO (de St. Groth, S. F. et al., J. Immunol. Methods (1980) 35, 1-21), S194 (Trowbridge, I. S. J. Exp. Med. (1978) 148, 313-323), and R210 (Galfre, G. et al., Nature (1979) 277, 131-133).

The above cell fusion of immunocytes with myeloma cells can be performed by a known method such as the method of Kohler, Milstein, and the like (Kohler. G. and Milstein, C., Methods Enzymol. (1981) 73, 3-46). Specifically, the above cell fusion can be performed in a general nutrient solution for culture in the presence of a cell fusion promoter, for example. As such a fusion promoter, polyethylene glycol (PEG), Sendai virus (HVJ: hemagglutinating virus of Japan), or the like is used, for example. An auxiliary agent such as dimethylsulfoxide can also be added and used to enhance cell fusion efficiency. Moreover, cell fusion can also be performed by an electric fusion method using an electroporation.

The mixing ratio of myeloma cells to immunocytes upon cell fusion is not limited and preferably ranges from 1:1 to 1:10. As a culture solution to be used for the above cell fusion, RPM11640 medium, MEM medium, or the like may be used, for example. A serum fluid (fluid replacement) such as fetal calf serum (FCS) may also be mixed therewith.

Cell fusion can be performed by mixing the above immunocytes and myeloma cells in medium, adding a PEG solution (e.g., the average molecular weight between approximately 1000 and 6000) previously heated at approximately 37°C to a concentration between 30% and 60% (w/v), and then mixing the cells and the solution, for example. Subsequently, a procedure comprising sequential addition of an appropriate culture solution and centrifugation to remove supernatants is repeated, so that a cell fusion agent and the like that are unfavorable for the growth of hybridomas are eliminated.

The thus obtained hybridomas are selected by culturing them in a general culture solution for selection, such as a HAT culture solution (the culture solution containing hypoxanthine, aminopterin, and thymidine). Culture in the above HAT culture solution is performed for a time length (generally, several days to several weeks) sufficient for cells other than the target hybridoma (unfused cell) to die. Subsequently, a general limiting dilution method is performed and then screening for and cloning of hybridomas producing the target antibody are performed. Screening can be performed by a known method such as ELISA or Western blotting.

The thus produced hybridomas producing a monoclonal antibody can be subcultured in a general culture solution or stored for a long time in liquid nitrogen.

A method that is employed to obtain a monoclonal antibody from such hybridomas is a method that involves culturing such hybridomas according to a general method and then obtaining the monoclonal antibody in a culture supernatant thereof, a method that involves administering such hybridomas to a mammal compatible therewith for the growth of the hybridomas and then obtaining the monoclonal antibody in an ascite, or the like. The former method is adequate for obtaining a highly-pure antibody, while the latter method is adequate for mass production of an antibody.

The present invention further encompasses a kit for producing an antigen-specific hybridoma. The kit contains a cytokine expression vector and stromal cells or stromal cells transfected with a cytokine expression vector, a polymeric biomaterial, and activated dendritic cells. The kit may further contain mice for artificial lymph node formation and immunodeficient mice. Moreover, the kit may further contain an antigen to be used to produce hybridomas. In this case, activated dendritic cells may be pulsed with the above antigen in advance.

The present invention is hereafter described in greater detail with reference to the following examples, although the present invention is not limited thereto.

Culture solutions, reagents, and instruments used in Examples are as follows.
RPMI-1640: GIBCO
2-mercaptoethanol: SIGMA
FCS (fetal calf serum): Cell Culture Technologies
Cell culture solution: RPMI-1640 supplemented with 10% inactivated FCS and 50 µM 2-mercaptoethanol
Trypsin-EDTA solution: GIBCO
G418 (geneticin): GIBCO
Recombinant mouse GM-CSF (granulocyte-macrophage colony stimulating factor): PeproTech
LPS (lipopolysaccharide): SIGMA
BSA (bovine serum albumin): SIGMA
Culture dish, culture plate, and Petri dish; all manufactured by FALCON
Preparation of artificial lymph nodes

Artificial lymph nodes were prepared in renal subcapsular spaces of Balb/c mice according to the method disclosed in JP Patent Publication (Kokai) No. 2004-255110 A. Specifically, artificial lymph nodes were prepared by the following method.

As stromal cells, TEL-2 cells (Nakashima M. et al., Eur J Immunol. 1990 Jan; 20(1): 47-53) established from the thymi of 2-week-old BALB/c mice were cultured in RPMI1640 supplemented with 10% fetal calf serum and 50 µM 2-mercaptoethanol. cDNAs of mouse lymphotoxin α (LTα) or chemokines (CCL21, CCL19, and CXCL13) were cloned from mouse spleen RNA by RT-PCR (reverse transcription-polymerase chain reaction). Each cDNA was inserted into the *EcoR* I site of a pCXN2 vector (Niwa H, et al., Gene. 1991 Dec 15; 108(2): 193-9). This vector contained a fowl β actin promoter, a CMV enhancer, and a rabbit β-globin splicing donor. TEL-2 cells were transfected with the thus obtained expression vector, so that stable transfectant cell lines were obtained after 2 weeks of G418 selection (500 µg/ml). After establishment of the stable transfectant cell lines, all cell lines were cultured in culture medium containing 200 µg/ml G418. Expression of the introduced gene was confirmed by analysis using a fluorescence-activated cell sorter (FACS) in the case of LTα and by chemotaxis assay in the case of the chemokines.

Collagen sponges ("Collagen Sponge" #CS-35, KOKEN, Tokyo, Japan) were cut into small pieces having a predetermined shape and size. Each collagen sponge piece was added to a well of a 48-well plate. Cytokine-producing TEL-2 stromal cells established as described above were collected using a Trypsin-EDTA solution. The collected cells were washed once with a culture solution, suspended in the culture solution, and then washed with PBS (phosphate buffered saline) once and then with 0.1% BSA/PBS once. One (1) ml of 1% BSA/PBS was added to each resultant so that cell suspensions were prepared. Each cell suspension was subjected to centrifugation, so that the cells were precipitated in the form of pellets. Cells were suspended in small amounts of 1% BSA/PBS. Thus, uniform cell suspensions with extremely high cell concentrations were prepared. Each cell suspension was added dropwise onto a collagen sponge piece and then the sponge was kneaded so that cells were adsorbed to the collagen sponge. Since cells were suspended in a small amount of 1% BSA/PBS solution and then the cells were adsorbed to collagen sponge pieces, we were careful not to dry the cells. A 48-well plate containing collagen sponge pieces to which the cells had been adsorbed was placed on ice until transplantation into mouse renal subcapsular spaces.

Medullary cavities of the femurs and tibias of 7- to 12-week old female BALB/cAnNCrj mice were flushed with PBS using a syringe provided with a 26-gauge injection needle, thereby resulting in bone marrow cell solutions. The bone marrow cell solutions were each filtered with nylon mesh to remove large cell masses and the like, so that a cell suspension adjusted to have a concentration of 2 × 10⁵cells/ml was prepared using the culture solution. In addition, the cell suspension contained erythroid cells at a high level; however, the cell concentration was calculated to be 2× 10⁵cells/ml with no regard to the number of such erythroid cells. The cell suspension was transferred at 7 ml per plastic dish to the plastic dish (Petri dish) having a diameter of 10 cm. Recombinant mouse GM-CSF was then added to a final concentration of 5 ng/ml. A half of the cell supernatant was discarded every 3 days or 4 days and then a new culture solution supplemented with 5 ng/ml GM-CSF was added. Floating (suspended) cells were collected on day 8 or 9 after initiation of culture. A cell suspension (2×10⁶ cells/ml) was prepared using a new culture solution supplemented with 5 ng/ml GM-CSF. When activation with the use of 1 µg/ml LPS (or TNF-α) and antigen pulsing were performed, the same antigen as the antigen to be used for inoculation into a mouse before transplantation of a polymeric biomaterial was added. Culture was performed for 17 to 20 hours using cell culture dishes, so that dendritic cells were caused to mature and become activated.

Collagen sponges ("Collagen Sponge" #CS-35, KOKEN, Tokyo, Japan) were cut into small pieces having a predetermined shape and size. Each collagen sponge piece was added to a well of a 48-well plate. Cytokine-producing TEL-2 stromal cells established as described above were collected using a Trypsin-EDTA solution. The collected cells were washed once with a culture solution, suspended in the culture solution, counted, and then placed on ice. The activated dendritic cells prepared by the above method were washed twice with a culture solution, suspended in the culture solution, counted, and then placed on ice. At this time, when dendritic cells were activated with LPS, the cells were thoroughly washed to avoid LPS from remaining. Subsequently, these cells were each washed once with PBS (phosphate buffered saline) and then washed once with 0.1% BSA/PBS. One (1) ml of 1% BSA/PBS was added to each resultant, so that cell suspensions having almost the same cell concentrations were prepared. These cell suspensions were mixed at a volume ratio of 1:1. Cells were precipitated in the form of pellets by centrifugation. A small amount of 1% BSA/PBS was further added, so that a uniform cell suspension having an extremely high cell concentration was prepared. The cell suspension was added dropwise onto each collagen sponge piece and then the collagen sponge piece was kneaded so that cells were adsorbed thereto. Since cells were suspended in a small amount of 1% BSA/PBS solution and then the cells were adsorbed to the collagen sponge pieces, we were careful not to dry the cells. A 48-well plate containing the collagen sponge pieces to which the cells had been adsorbed was maintained on ice until transplantation thereof into mouse renal subcapsular spaces.

Eight-to-forteen-week-old female BALB/cAnNCrj mice (Charles River Laboratories Japan, maintained in a mouse rearing room under an SPF environment) were anesthetized. Body surfaces were disinfected with 70% ethanol and the mice were placed in the right lateral decubitus position. An approximately-1-cm incision was made on the skin of left hypochondrium and then an incision of almost the same size as that of the above incision was made on the muscle layer immediately below the incised part. Adipose tissue in the periphery of the kidney was anchored with forceps, so that the kidney was excised from the body. The renicapsule was opened using forceps with sharp ends, while carrying out observation under a stereomicroscope so as not to damage the renal parenchyma. Collagen sponge pieces were inserted between the renicapsule and the kidney. In general, for transplantation of collagen sponge pieces to two sites per kidney (the position in the vicinity of superior pole of the kidney and the position in the vicinity of inferior pole of the kidney), 4 collagen sponge pieces to which stromal cells had been adhered were transplanted into the left and the right kidneys of each mouse.

Recipient BALB/cAnNCrj mice were immunized 4 weeks before transplantation via intraperitoneal administration of the antigen prepared by precipitation of NP-ovalbumin (NP-OVA) in alum.

Artificial lymph nodes were collected 3 weeks after transplantation. The thus collected artificial lymph nodes were transplanted into renal subcapsular spaces of SCID mice (C.B-17/IcrCrj-scid/scid).

On the next day, SCID mice were immunized with NP-OVA. At this time, NP-OVA was dissolved in PBS, and then 100 µg of the antigen was intravenously administered. Further, after 1 week, booster immunization was performed using the same antigen. The amount of the antigen administered herein was 10 µg, which was one-tenth of the first amount thereof.

After second immunization, sera were collected and the spleens were excised from SCID mice on days 4 to 5. The thus collected sera were subjected to measurement of the concentrations of IgG anti-NP antibody therein. Furthermore, the numbers of antibody-producing cells in the spleens were counted by the ELISpot method. Moreover, spleen cells were prepared and then cell suspensions were prepared using cell culture medium. In addition, the steps of artificial lymph node construction, retransplantation of artificial lymph nodes into SCID mice, and immunization of SCID mice were conducted under varied conditions, including with the presence and the absence of antigen-pulsing of dendritic cells to be used for artificial lymph node construction and the presence and the absence of primary immunization with 100 µg of the antigen and secondary immunization with 10 µg of the antigen. Furthermore, splenocytes were collected from immune mice including naive Balb/c mice, naive SCID mice, Balb/c mice (such Balb/c mice having been preimmunized with the antigen) reimmunized twice intravenously using the antigen, and Balb/c mice preimmunized with the antigen. These splenocytes (1×10⁷ cells) were transplanted into SCID mice via intravenous injection. Subsequently, SCID mice reimmunized twice with the antigen were used as control mice.

Table 1 shows serum anti-NP antibody concentrations for Balb/c mice subjected to secondary immunization without retransplantation of artificial lymph nodes, SCID mice subjected to retransplantation of artificial lymph nodes and secondary immunization (the method of the present invention), SCID mice subjected to retransplantation of artificial lymph nodes but not subjected to immunization thereafter, and SCID mice subjected to secondary immunization without retransplantation of artificial lymph nodes.

**Table 1**

| Serum IgG1 class anti-NP antibody | |
|---|---|
| Serum IgG1 class anti-NP antibody (µg/ml) | |
| Balb/c, preimmunized (after secondary immunization) | 673.3 ± 271.8 |
| SCID having artificial lymph nodes (after secondary immunization) | 7,258.9 ± 707.9 |
| SCID having artificial lymph nodes (no immunization) | 0.61 ± 0.32 |
| SCID (after secondary immunization) | < 0.001 |

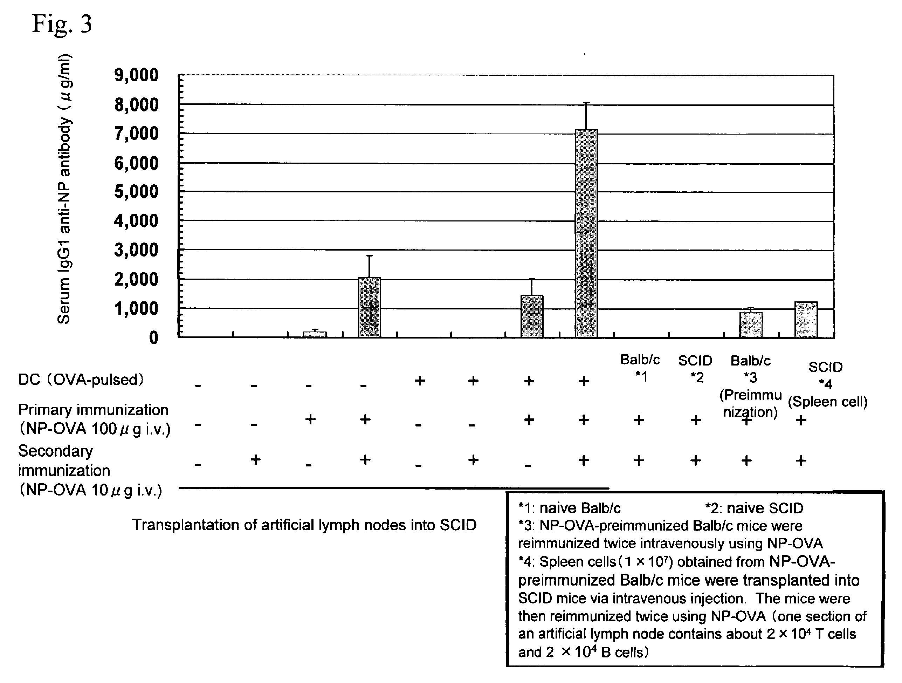

As shown in Table 1, the sera of SCID mice subjected to transplantation of artificial lymph nodes and immunization with the antigen contained at high levels the high-affinity antibody (in this case, the antibody against NP6) having high affinity for the antigen. In the Example, 7 mg/ml antigen-specific IgG class antibody was contained. It can be understood that a high level of the antibody was produced since the general total immunoglobulin level in a mouse serum is 1 mg/ml. Fig. 3 shows serum IgG antibody levels of SCID mice immunized under each condition. As is understood from Fig. 3, it is important for dendritic cells to be pulsed with an antigen in advance and is also important for immunization to be performed twice intravenously. Moreover, Fig. 3 shows that no effects can be obtained by simple intravenous administration of normal lymph nodes that have been subjected to high-level antigen stimulation to SCID mice, followed by similar immunization of the SCID mice with the antigen. Fig. 3 also shows that such an effect cannot be obtained unless artificial lymph nodes are used.

Table 2 shows the numbers of cells producing IgG class anti-NP specific antibody in the spleens of SCID mice subjected to retransplantation of artificial lymph nodes.

**Table 2**

| Numbers of cells producing the IgG class NP-specific antibody in the spleens of SCID mice having artificial lymph nodes after antigen challenge | | | |
|---|---|---|---|
| | Numbers of cells producing the IgG class anti-NP antibody after reimmunization (per 10⁵ cells) | | |
| | IgG1 (NP6) | IgG1 (NP30) | IgM (NP30) |
| SCID mice (artificial lymph nodes transplanted) (approximately 8 × 10⁴ cells) (no immunization) | 7 ± 4 | 34 ± 15 | 302 ± 77 |
| SCID mice (artificial lymph nodes) (approximately 8 × 10⁴ cells) (immunized twice intravenously using NP-OVA) | 31,496 ± 6,774 | 37,744 ± 6,936 | 2,384 ± 242 |
| Artificial lymph nodes in SCID mice (immunized twice intravenously using NP-OVA) | 1,046 ± 126 | 991 ± 182 | 261 ± 120 |

As shown in Table 2, when spleen cells of SCID mice immunized (subjected to transplantation of artificial lymph nodes) according to the method of the present invention were examined, 30,000 or more cells (approximately 30% or more) producing (NP) antigen-specific IgG class antibody were present among 100,000 spleen cells. That is, an organism can be produced by the method of the present invention in which at least one out of every three or four lymphocytes (spleen cells) are cells producing an antigen-specific antibody.

Spleen cells and myeloma cells (myeloma) were mixed at a ratio of 10:1 and then caused to undergo cell fusion using polyethylene glycol 1500 (produced by Boehringer Mannheim) as a fusion agent, so that numerous hybridomas were prepared. Myeloma cells P3X63Ag8U.1 were used. Hybridomas were selected by culturing them in HAT-containing DMEM medium (produced by Gibco BRL) containing 10% fetal calf serum (FCS), hypoxanthine (H), aminopterin (A), and thymidine (T). Furthermore, single clones were obtained by the limiting dilution method using HT-containing DMEM medium. Culture was performed in 96-well microtiter plates, such that 10⁴ cells were seeded in each well and then cultured. Screening for hybridoma clones producing the anti-NP monoclonal antibody was performed by enzyme-linked immunosorbent assay (ELISA). At this time, horseradish-peroxidase-labeled anti-mouse IgG antibody was used as a secondary antibody, and the absorbance of each well was measured after enzyme reaction using an ELISA reader.

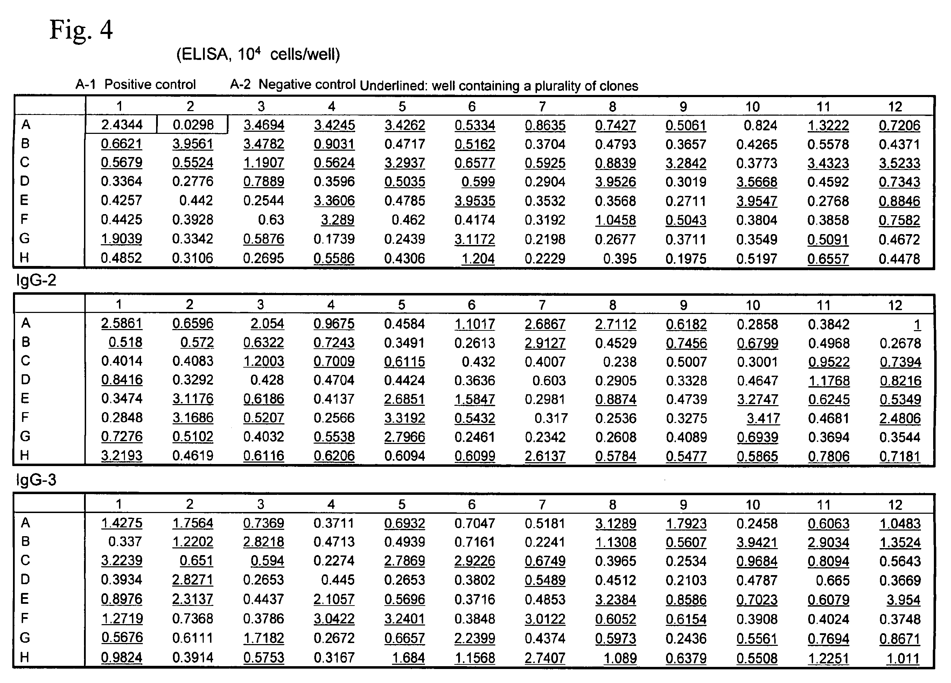

Fig. 4 shows the results of ELISA measurement for each well. As shown in Fig. 4, when cell fusion with mouse myeloma cells had been performed by the method of the present invention, almost all hybridomas that could be obtained herein produced the antigen-specific antibody.

### Industrial Applicability

Artificial lymph nodes that are used in the method of the present invention can rapidly induce a high level of antigen-specific immunoreaction and can efficiently supply effective antibody-producing cells. Through production of hybridomas using such artificial lymph nodes, antigen-specific hybridomas can be produced at high formation rates. In particular, the artificial lymph node of the present invention is advantageous for use in production of a hybridoma specific to an antigen with low immunogenicity, for example.

All publications, patents, and patent applications cited in this description are herein incorporated by reference in their entirety.

## Claims

1. A method for production of an antigen-specific hybridoma, comprising the following steps of:
(a) immunizing a nonhuman animal with an antigen and then transplanting a polymeric biomaterial containing a stromal cell that produces a cytokine into the immunized nonhuman animal, thereby causing the nonhuman animal to form an artificial lymph node;
(b) collecting the artificial lymph node formed in step (a) and then transplanting the artificial lymph node into a nonhuman immunodeficient animal of the same species as the nonhuman animal in step (a);
(c) immunizing the nonhuman immunodeficient animal into which the artificial lymph node is transplanted in step (b) with the antigen of step (a);
(d) collecting the splenocytes from the nonhuman immunodeficient animal immunized in step (c); and
(e) fusing the splenocyte collected in step (d) with a myeloma cell in order to make an antibody-producing hybridoma.

2. The method of according to claim 1, in which the polymeric material further contains an activated dendritic cell pulsed with the antigen of step (a) in claim 1.

3. The method according to claim 1 or 2, in which the stromal cell is a TEL-2 cell.

4. The method according to any one of claims 1 to 3, in which the polymeric material is a collagen sponge.

5. The method according to any one of claims 1 to 4, in which the cytokine is lymphotoxin and/or chemokine.

6. The method according to any one of claims 1 to 5, in which the nonhuman animal is a mouse.

7. The method according to any one of claims 1 to 6, in which the immunodeficient animal is an immunodeficient mouse.

8. The method according to any one of claims 1 to 7, in which the immunodeficient mouse is an SCID mouse or a RAG2 gene-deficient mouse.

9. A kit for producing an antigen-specific hybridoma, comprising:
(a) a cytokine expression vector and a stromal cell or a stromal cell transfected with a cytokine expression vector;
(b) a polymeric biomaterial; and
(c) an activated dendritic cell.

10. The kit for producing an antigen-specific hybridoma according to claim 9, further comprising:
(d) a mouse for artificial lymph node formation; and
(e) an immunodeficient mouse.

11. The kit for producing an antigen-specific hybridoma according to claim 9 or 10, in which an antigen is further contained and the activated dendritic cell is pulsed with the antigen.
